# FASCICULE DE BREVET EUROPEEN

(11) **EP 1 912 513 B1**
(45) Date de publication et mention de la délivrance du brevet: **11.09.2013**
(21) Numéro de dépôt: 06794209.4
(22) Date de dépôt: 25.07.2006
(51) Int. Cl.: A23C 9/146, A61K 38/40, A61K 38/18, A61K 38/30, A61K 39/395, A61K 35/20, A61P 29/00

(54) **NOUVELLES FRACTIONS PROTEIQUES LAITIERES ET LEUR UTILISATION POUR LA PREVENTION OU LE TRAITEMENT DES MALADIES INFLAMMATOIRES CHRONIQUES**
NEUE MILCHPROTEINFRAKTIONEN UND IHRE VERWENDUNG FÜR DIE VORBEUGUNG ODER BEHANDLUNG VON CHRONISCHEN ENTZÜNDUNGSKRANKHEITEN
NOVEL MILK PROTEIN FRACTIONS AND USE THEREOF FOR PREVENTING OR TREATING CHRONIC INFLAMMATORY DISEASES

(30) Priorité: 29.07.2005 FR 0508177
(43) Date de publication de la demande: 23.04.2008
(73) Titulaire: COMPAGNIE LAITIERE EUROPEENNE, F-50890 Condé-sur-Vire (FR)
(72) Inventeur: MIKOGAMI, Takashi, F-35300 Fougeres (FR); SOUPPE, Jérôme, F-35700 Rennes (FR); JOUAN, Pierre, F-35510 Cesson Sevigne (FR); BOURTOURAULT, Michel, F-35230 Noyal Chatillon Sur Seiche (FR)
(74) Mandataire: Corizzi, Valérie
(86) Numéro de dépôt international: PCT/FR2006/001810
(87) Numéro de publication internationale: WO 2007/012748

(56) Documents cités:
- WO-A-01/25276
- WO-A-03/008447
- FR-A- 2 841 747
- US-A- 5 149 647
- US-A1- 2003 059 477
- HAHN R ET AL: "Bovine whey fractionation based on cation-exchange chromatography" JOURNAL OF CHROMATOGRAPHY A, ELSEVIER, AMSTERDAM, NL, vol. 795, no. 2, 6 février 1998 (1998-02-06), pages 277-287, XP004460478 ISSN: 0021-9673

## Description

La présente invention a pour objet de nouvelles fractions protéiques laitières enrichies en TGF-β, un procédé pour leur préparation et leur utilisation pour la préparation d'un médicament et/ou une composition alimentaire destiné à la prévention et/ou au traitement de maladies inflammatoires chroniques, et en particulier du psoriasis.

Le psoriasis est une affection dermatologique chronique, caractérisée par une éruption érythémato-squameuse, qui évolue par poussées, de façon prédominante au niveau des coudes, des genoux et du cuir chevelu.

Du point de vue biologique, le psoriasis est un processus inflammatoire chronique qui se caractérise par une prolifération et une différentiation anormales des kéranocytes associées à une infiltration du derme et de l'épiderme par des lymphocytes T et des polynucléaires neutrophiles qui forment des micro-abcès dans la couche cornée.

Les causes de l'apparition du psoriasis chez un individu sont mal connues. Plusieurs facteurs favorisant l'apparition du psoriasis sont cités :
- un facteur héréditaire,
- un facteur psychologique (stress, changements hormonaux, ...),
- une anomalie immunitaire.

Enfin certains médicaments et les infections bactériennes ou virales sont susceptibles de déclencher des crises de psoriasis.

A l'heure actuelle, aucun traitement n'est capable de guérir le psoriasis. Les traitements connus à ce jour ne peuvent que suspendre et/ou atténuer les symptômes du psoriasis.

Pour les psoriasis limités à quelques plaques, on prescrit de la vitamine D, éventuellement associée à des corticoïdes en application locale. On peut également prescrire des rétinoïdes en application topique.

Dans les cas les plus graves on prescrit de la photothérapie, ou éventuellement du méthotrexate ou des rétinoïdes par voie générale. Ces derniers traitements sont associés à des effets secondaires importants.

Il n'existe donc pas à l'heure actuelle de traitement satisfaisant du psoriasis.

Les autres maladies inflammatoires chroniques, telles que l'arthrite rhumatoïde, l'ostéoarthrite, la maladie de Crohn, la sclérose en plaques, le lupus érythémateux, posent les mêmes problèmes au praticien : il n'existe pas de traitement permettant de guérir ces pathologies et les traitements existants pour traiter les manifestations de ces pathologies sont soit insuffisants, soit associés à des effets secondaires très lourds. Une composante inflammatoire chronique est également présente dans les maladies auto-immunes.

On sait que les facteurs de croissance, de la même façon que les chimiokines et les cytokines, produisent des effets sur les processus inflammatoires. Ces effets vont de l'amplification à la suppression du processus inflammatoire.

On sait notamment que certains facteurs de croissance du lait ont une activité modulatrice du processus inflammatoire.

Par exemple, le document WO 96/34614 décrit l'utilisation d'un produit lacté pour la préparation d'un médicament pour la prévention d'une lésion de la muqueuse du tube digestif provoquée par une chimiothérapie ou une radiothérapie.

L'extrait est préférentiellement obtenu par passage du lait sur une colonne de chromatographie échangeuse de cations. Il comprend de préférence de la lactoferrine et/ou de la lactoperoxydase et un facteur de croissance, notamment le TGF-β.

Le TUF-β (Transforming Growth Factor) est un facteur de croissance peptidique qui régule la croissance et la différentiation cellulaire. Il s'agit d'une molécule dimérique de 25 kD qui peut se présenter sous différentes isoformes : TGFß₁, TGFß₂ et TGFß₃. Le TGF-β est connu pour sa capacité à réguler, et notamment à réduire certaines étapes de la réaction immunitaire et inflammatoire (G. Prud'homme et al., J. Autoimmun. (2000), 14, 23-42 ; M. Shull et al., Nature (1992), 359, 693-699 ; J.Graycar et al., Mol. Endocrinol. (1989), 3, 1977-1986 ; J.Letterio et al., Annu. Rev. Immunol. (1998), 16, 137-161). Il est présent sous forme latente (non active biologiquement) ou sous forme active.

Il est présent dans le lait en très petite quantité (30 µg/l de lait). Toutefois, dans le lait sont présents de nombreux autres facteurs ayant une activité différente de celle du TGF-β, éventuellement opposée à celle du TGF-β, et la conséquence en est que le TUF-β contenu dans le lait n'a pas d'action notable sur le processus inflammatoire.

Par ailleurs, l'isolement du TGF-ß à partir du lait est peu réalisable à l'échelle industrielle, tout d'abord pour des raisons de coût et également parce que les procédés d'isolement entraînent souvent une dénaturation des protéines visées et conduisent alors à l'obtention d'une fraction protéique laitière ayant perdu toutes les propriétés biologiques des protéines qu'elle contient.

Le document EP 0 313 515 décrit un procédé d'isolement du TGF-β à partir du lait. Toutefois, ce procédé comporte de très nombreuses étapes, à caractère complexe, et reste donc peu viable sur le plan économique.

Dans le but d'obtenir une fraction protéique laitière qui montre une activité biologique sur les processus inflammatoires, c'est-à-dire une réduction ou une suppression des processus inflammatoires, il faudrait pouvoir, à partir du lait, augmenter la teneur en TGF-β par rapport aux autres protéines, tout en éliminant au moins certains des facteurs antagonistes du TGF-β qui bloquent ses propriétés anti-inflammatoires lorsqu'il est dans le lait. La difficulté de cette démarche est d'autant plus grande que l'on ignore quels sont exactement les antagonistes du TUF-β.

Plusieurs auteurs se sont penchés sur le problème de l'isolement de fractions protéiques laitières enrichies en TGF-β. C'est le cas des demandes de brevets et brevets WO 96/34614, EP 0 545 946, WO 01/25276, WO 03/008447, FR 2 827 240, EP 0869 134.

Le document EP-0 545 946 décrit un procédé d'extraction de facteurs de croissance du lactosérum ; ce procédé comprenant les étapes de : filtration du sérum pour retirer les insolubles, ajustement du pH entre 6,5 et 8, utilisation d'une résine échangeuse de cations de type Sépharose, sur laquelle les principaux constituants basiques sont adsorbés et les principales protéines acides sont éluées, équilibrage de la résine par un tampon, application du filtrat sur la résine, élution avec un tampon de force ionique élevée, filtration pour éliminer les sels, concentration.

Le document EP 0 869 134 décrit un procédé pour récupérer un ou plusieurs facteurs de croissance à partir de lait ou d'un dérivé du lait par adsorption sur un échangeur de cations, élution fractionnée, obtention d'une fraction enrichie en facteurs de croissance, puis traitement à un pH allant de 3,5 à 4,5. Le procédé est appliqué de préférence avec une vitesse superficielle élevée et une charge de liquide élevée.

Le document WO 01/25276 décrit un procédé d'extraction du TGF-β et de l'IGF-1 comprenant les étapes suivantes : récupération d'une fraction basique du lait par chromatographie échangeuse de cations, passage des fractions récupérées sur une colonne d'hydroxyapatite, élution avec au moins deux éluants de concentration croissante en sels de façon à obtenir deux fractions :
- une fraction enrichie en IGF-1 avec IGF-1/TGF-β > 10
- une fraction enrichie en TUF-β avec TGF-β/IGF-1 > 5 et qui contient de 30 à 50 % d'immunoglobulines par rapport à la quantité totale de protéines.

Le document FR 2 827 240 décrit un procédé d'obtention d'une fraction protéique enrichie en TGF-β sous forme activée, à partir d'une solution riche en protéines solubles de la phase aqueuse du lait par : a) ajustement de la concentration en protéines solubles à 5-30 g/l, b) précipitation par traitement acide, c) microfiltration-diafiltration, d) récupération du rétentat de microfiltration, e) séchage.

Le document WO 03/008447 décrit un procédé pour isoler les facteurs de croissance TGF-ß, IGF-1, la lactoperoxydase et des immunoglobulines. Une fraction protéique basique du lait est récupérée par chromatographie d'échange cationique. La fraction obtenue est passée sur chromatographie d'interaction hydrophobe.

Toutefois, aucun de ces procédés n'a permis d'obtenir dans des conditions industriellement viables, une fraction protéique laitière ayant une réelle efficacité sur les pathologies inflammatoires chroniques et sur leurs manifestations ou symptômes, et notamment sur le psoriasis.

Ainsi, c'est avec étonnement que la Demanderesse a découvert de nouvelles fractions protéiques laitières ayant une action sur les processus inflammatoires chroniques et notamment sur le psoriasis.

Les fractions protéiques laitières de l'invention sont susceptibles d'être obtenues par un procédé comprenant les étapes suivantes :

On utilise du lait ou du lactosérum comme produit de départ.
(a) le lait ou le lactosérum est éventuellement soumis à une microfiltration ou à un traitement thermique ;
(b) le lait ou le lactosérum ou le produit issu de l'étape (a) est déposé sur une résine échangeuse de cations constituée d'un polymère synthétique nanoporeux fonctionnalisé des groupes fonctionnels de type base conjuguée d'un anion d'un acide fort ; la vitesse linéaire est comprise entre 2,8 et 4,5 m/h et le volume de lait ou de lactoserum par rapport au volume de résine est compris entre 100 et 200;
(c) la résine échangeuse de cations est lavée par de l'eau déminéralisée ;
(d) la résine est éluée par des solutions de saumure de concentration croissante ;
(e) un éluat correspondant à une solution de saumure de conductivité comprise entre 21,0 et 22,0 mS/cm est récupéré.

Le procédé de l'invention peut en outre encore comporter une ou plusieurs des étapes suivantes :
(f) l'éluat obtenu en (e) est concentré par ultrafiltration et on récupère le rétentat ;
(g) le rétentat obtenu en (f) est stérilisé par microfiltration et on récupère le perméat ;
(h) le perméat obtenu en (g) est séché par atomisation.

On peut utiliser comme produit de départ dans le procédé selon l'invention soit du lait, soit du lactosérum, préférentiellement issus de la vache. Le lactosérum est le liquide résiduel obtenu après l'extraction des protéines et de la matière grasse du lait ou du petit-lait. On distingue en général trois catégories de lactosérum. Les deux premières catégories sont classées selon l'acidité du lactosérum qui peut être inférieure ou supérieure à 1,8 g d'acide lactique/l : le lactosérum doux, issu de la fabrication de fromage à pâte pressée cuite ou non cuite (emmenthal, saint-paulin etc.) et le lactosérum acide, issu de la caséine ou d'autres fromages obtenus par coagulation mixte ou lactique (pâtes molles, pâtes fraîches). La composition moyenne du lactosérum doux est à titre indicatif, pour 61 g de matières sèches par kg de lactosérum, de 42 à 48 g de lactose, 8 g de protéines, 2 g de graisses, 5 à 7 g de minéraux, 1 à 5 g d'acide lactique et le reste en minéraux et vitamines.

On connaît également le lactosérum idéal obtenu par microfiltration du lait sur un support de porosité moyenne de 0,1 µm.

Selon une première variante de l'Invention, on utilise comme produit de départ du lait, et avantageusement du lait de vache, dont la composition permet, par le procédé selon l'invention, l'obtention d'un isolat de protéines ayant des propriétés biologiques plus intéressantes. Cette variante permet également d'obtenir un rendement en protéines supérieur aux variantes utilisant le lactosérum comme produit de départ.

Selon une seconde variante de l'Invention, on utilise comme produit de départ du lactosérum acide de caséinerie. Cette variante représente un avantage économique dans la mesure où le produit de départ est un sous-produit issu de l'exploitation industrielle et donc d'un faible coût.

Le lait utilisé comme produit de départ peut être du lait de vache, de chèvre, de brebis, de bufflonne. Il peut être dégraissé de façon connue par centrifugation.

Le lait ou le lactosérum est soumis à un traitement qui permet d'éliminer toute contamination d'origine microbienne. Dans ce but, le lait est avantageusement soumis à un traitement thermique, à une température ne dépassant pas 68°C, ou à une première étape de microfiltration, par exemple sur un filtre de porosité d'environ 1,4 µm.

Puis le perméat de microfiltration, ou directement le lait ou le lactosérum, est déposé sur la colonne de résine.

La résine utilisée dans le procédé décrit ci-dessus est constituée d'un polymère synthétique nanoporeux polyanionique. Le polymère est de préférence tridimensionnel de forme sphérique ou sphéroïdale. Il est porteur de groupes fonctionnels de type acide fort, sous forme de la base conjuguée d'un anion de cet acide fort. Il est préférentiellement fonctionnalisé par des groupes SO₃⁻. En outre, le polymère dont est constitué la résine doit posséder des propriétés de résistance mécanique lui permettant de résister aux contraintes hydrauliques résultant des paramètres d'élution préférés.

A l'étape (b), le débit d'alimentation de la colonne est compris entre 10 et 15 m³/h et la vitesse linéaire est comprise entre 2,8 et 4,5 m/h.

A l'étape (b), le rapport du volume de lait au volume de résine est compris entre 100 et 200.

Après le dépôt du lait ou du lactosérum, la colonne de résine est lavée par de l'eau déminéralisée, en utilisant de préférence 10 à 151 d'eau/l de résine.

On utilise ensuite une solution de saumure comprenant une concentration de sel de 10 à 14 g/l, de préférence de 11 à 13 g/l.

Le débit de saumure est de 1,5 à 2m³/h et la vitesse linéaire de la solution de saumure est avantageusement de 0,4 à 0,6 m/h. Le volume de saumure par rapport au volume de résine est compris entre 2,5 et 3,5.

L'éluat ainsi récupéré est ensuite concentré par une ou plusieurs étapes d'ultrafiltration, et éventuellement une ou plusieurs étapes de diafiltration. Ces étapes se font avantageusement à basse température (2 à 6° C).

Le rétentat ainsi obtenu est microfiltré et séché par atomisation.

D'autres méthodes de déminéralisation et de concentration connues de l'homme du métier peuvent toutefois être envisagées.

On obtient ainsi une fraction protéique laitière ayant les caractéristiques suivantes :
- teneur en TGF-β comprise entre 0,010 et 0,025 % en poids par rapport au poids total des protéines.
- teneur en IgG (Immuno Globuline) < 25 % en poids par rapport au poids total des protéines ;
- ratio TUF-β / IGF 1 ≥ 5 en poids/poids.
- teneur en lactoperoxydase comprise entre 35 et 45 % en poids par rapport au poids total des protéines ;

Ces fractions protéiques laitières constituent un autre objet de l'invention.

De façon étonnante, les fraction protéiques laitières de l'invention provoquent une réduction de la prolifération lymphocytaire telle qu'évaluée sur des cellules de rate de souris sacrifiées : les cellules traitées au chlorure d'ammonium et lavées sont mises en incubation pendant 48 heures en présence des fractions protéiques objets de l'invention et d'un agent mitogène comme la concanavaline A avec ajout de 5-bromodéoxyuridine dans le milieu cellulaire en fin de culture. La prolifération lymphocytaire est évaluée par la quantité de 5-bromodéoxyuridine incorporée dans les cellules.

Sachant que l'épiderme psoriasique est le siège d'un afflux de lymphocytes T activés, les fractions protéiques, objet de l'invention, peuvent être utilisées pour la prévention ou le traitement du psoriasis, mais aussi d'autres affections telles que les pathologies inflammatoires chroniques et/ou leurs symptômes. Parmi ces pathologies on peut citer le psoriasis mais aussi l'arthrite rhumatoïde, l'ostéoarthrite, la maladie de Crohn, la sclérose en plaques, le lupus érythémateux. En outre, elles sont susceptibles d'avoir un effet sur la composante inflammatoire des maladies auto-immunes. Elles sont donc utilisables pour la prévention et/ou le traitement des maladies auto-immunes, en particulier pour la prévention et/ou le traitement de la composante inflammatoire des maladies auto-immunes.

En outre, le procédé de l'invention est facilement réalisable à l'échelle industrielle, sa mise en oeuvre étant simple et ne comportant qu'un nombre limité d'étapes.

Un autre avantage du procédé de l'invention réside dans le fait qu'il permet de récupérer d'autres fractions protéiques laitières qui présentent un intérêt industriel certain. On peut éluer la colonne par des solutions aqueuses de saumure de concentration croissante qui permettent de récupérer différentes fractions protéiques laitières, éventuellement après avoir récupéré la fraction de l'étape e). Dans une étape e') la fraction qui est éluée avec une solution de saumure de conductivité allant de 50,5 à 51,5 mS/cm est une fraction enrichie en lactoferrine. Elle permet de récupérer une composition protéique laitière riche en lactoferrine en une seule étape de chromatographie. De préférence cette étape de procédé est mise en oeuvre en utilisant une ou plusieurs des conditions suivantes :
- volume de saumure par rapport au volume de résine compris entre 2,5 et 3,5,
- débit de saumure compris entre 1,5 et 2 m³/h,
- vitesse linéaire d'élution comprises entre 0,4 et 0,6m/h.

Il peut en outre comporter des étapes ultérieures, d'ultrafiltration, de diafiltration, de microfiltratio et/ou de séchage, notamment par atomisation.

Ce procédé d'isolement d'une fraction protéique laitière enrichie en lactoferrine constitue un autre objet de l'invention.

Un autre objet de l'invention est une composition pharmaceutique comprenant une fraction protéique laitière de l'invention et un support pharmaceutiquement acceptable.

En fonction de la pathologie concernée et de la gravité de cette pathologie, le mode d'administration et le support sera adapté par l'homme du métier : application topique (crème, lotion, patch dermique), administration orale (gélules, sirop, comprimé, solution ou dispersion aqueuse), injection (solution injectable).

On peut également prévoir d'utiliser les fractions protéiques laitières de l'invention pour la préparation de compositions alimentaires, notamment de compositions diététiques, destinées plus particulièrement aux personnes atteintes de pathologies inflammatoires chroniques, et notamment de psoriasis, ou de maladies auto-immunes. De telles compositions alimentaires comportent une fraction protéique laitière de l'invention en remplacement des protéines de lait habituellement employées. Elles peuvent être des boissons protéiques, des aliments lactés...Elles constituent un autre objet de l'invention.

La quantité de fraction protéique laitière à administrer et la fréquence d'administration sont adaptées par l'homme du métier en fonction de la pathologie, de sa gravité, de l'âge et du poids du patient.

Les compositions pharmaceutiques ou diététiques de l'invention peuvent être utilisées pour le traitement d'une pathologie inflammatoire chronique, en particulier du psoriasis, ou d'une maladie auto-immune, en période de crise. Elles peuvent également être employées en période de rémission de façon à prévenir et/ou éviter et/ou espacer l'apparition de nouvelles périodes d'atteinte aiguë.

### EXEMPLE 1 : Préparation d'une fraction protéique laitière

250 m³ de lait écrémé et préalablement traité thermiquement à 68°C pendant 15 secondes sont assujettis à passer dans une colonne de chromatographie (dans le sens descendant) sur 20001 de résine échangeuse de cations (SPEC 70 fournie par BIOSEPRA) ayant les caractéristiques suivantes :

Polymère totalement synthétique macroporeux polyanionique fonctionnalisé par des groupements SO₃⁻ ; le polymère est tridimensionnel, de forme sphérique ou sphéroïdale ; la taille des particules est supérieure à 261µm ; le support est fabrique par polymérisation de l'AMPS : acide 2-acrylamide 2-méthyl propane sulfonique.

Le débit d'alimentation de la colonne en lait est de 14 m³/h et la vitesse linéaire de 3,2m/h.

Après passage du lait, la colonne est lavée par 23 000 litres d'eau déminéralisée (dans le sens descendant).

Une solution de saumure à 12g/l est ensuite utilisée pour extraire de la résine les protéines fixées. Le débit utilisé est de 1900 1/h et la vitesse linéaire de 0,6m/h ; le volume d'éluat obtenu est proche de 8 000 litres.

Cet éluat est ensuite concentré par UF (DSS, membranes GR10D 400m) à 4° C en pratiquant un facteur de concentration volumique tel que la conductivité du rétentat reste supérieure à 15mS/cm. Le rétentat obtenu est ensuite concentré une seconde fois par UF et diafiltration (équipé des mêmes membranes mais avec une surface de seulement 34m²) jusqu'à obtenir une conductivité du rétentat de 5 mS/cm.

Le rétentat ainsi obtenu est passé à 30° C sur un module de microfiltration de 17m² et de porosité de 1,4µm ; le perméat obtenu présente un extrait sec proche de 8% et est atomisé sur une tour à buse simple effet avec une température d'entrée d'air de 180° C et une température de sortie de tour de 80° C.

On récupère 1650g de fraction protéique laitière sous forme de poudre. Cette fraction présente les caractéristiques suivantes :

| | |
|---|---|
| Humidité | 5,4% |
| Protéines | 94,2% |
| Cendres | 0,9% |
| Teneur en TGF-β | 110µg/g de protéines |
| Teneur en lactoperoxydase | 407mg/g de protéines |
| Teneur en IgG | <190mg/g de protéines |

### EXEMPLE 2 : Effet sur la croissance cellulaire de la fraction protéique laitière

L'effet de cette fraction protéique laitière obtenue dans l'Exemple 1 a été testé sur une culture de cellules provenant d'une effusion pleurale d'un adénocarcinome du sein, MCF7. Deux expériences sont réalisées. Les cellules MCF7 sont ensemencées dans des boîtes de 25 cm² à la concentration de 700 000 cellules/ boîte pour l'Expérience 1 et de 200 000 cellules/boîte pour l'Expérience 2. Après 24 heures d'attente, le milieu est aspiré et remplacé par 7 ml de milieu contenant la fraction protéique obtenue dans l'Exemple 1 à la concentration de 218 µg/ml, 109 µg/ml ou 0 µg/ml (milieu témoin). Puis les cellules sont cultivées pendant 72 heures avec le changement de milieu toutes les 24 heures. Les résultats correspondent à la moyenne des comptages réalisés sur 4 boîtes; pour chaque essai.

Les expériences démontrent bien un effet inhibiteur de façon dose dépendante de la fraction protéique laitière obtenue dans l'Exemple 1 sur la croissance des cellules MCF7.

| Concentration de la fraction protéique dans le milieu (µg/ml) | | 218 | 109 | 0 (témoin) |
|---|---|---|---|---|
| Expérience 1 | Nombres de cellules (10⁶) | 3,64 ± 0,46 | 4,64 ± 0,40 | 6,74 ± 0,70 |
| | % de baisse par rapport au témoin | 46,0% | 31,2% | |
| Expérience 2 | Nombres de cellules (10⁶) | 0,87 ± 0,05 | 1,15 ± 0,08 | 1,67 ± 0,08 |
| | % de baisse par rapport au témoin | 47,9% | 31,1% | |

### EXEMPLE 3 : Préparation d'un complément nutritionnel comprenant la fraction protéique laitière

Un complément alimentaire sous forme de gélule a été préparé en incorporant la fraction protéique laitière obtenue dans l'Exemple 1.

| | Pour 1 gélule de 350 mg |
|---|---|
| La fraction protéique laitière obtenue dans l'Exemple 1 | 125 mg |
| Omégacaps^{®} de la société Polaris (Poudre d'huile de poisson contenant 20% d'acide gras oméga 3) | 125 mg (dont 25 mg d'oméga 3) |
| Vitamine PP (niacine) | 1,25 mg |
| Stéarate de magnésium | 5 mg |
| Emeocel^{®} | 93,75 mg |

### EXEMPLE 4 : Effets du complément nutritionnel comprenant la fraction protéique laitière sur les personnes atteintes du psoriasis

Les effets de ce complément nutritionnel préparé dans l'Exemple 3 ont été testés. Vingt personnes (8 femmes et 12 hommes âgés de 23 à 70 ans) qui sont atteints du psoriasis ont ingéré quotidiennement 8 gélules (4 gélules le matin et 4 gélules le soir) du complément nutritionnel préparé dans l'Exemple 3 pendant 60 jours. C'est-à-dire, l'ingestion journalière de la fraction protéique laitière obtenue dans l'Exemple 1 était de 1 g, ce qui correspond à 110 µg de TGF-β Les contrôles symptomatiques et les analyses sanguines ont été réalisés avant et après 60 jours de l'ingestion ce complément nutritionnel.

### Résultats :

- Symptômes psoriasiques : après 60 jours de l'ingestion de ce complément nutritionnel, pour 80% de personnes, une nette amélioration des symptômes (diminutions de plaques psoriasiques, de l'inflammation, de la desquamation et du prurit) a été observée.

| Évaluation globale de symptômes psoriasiques | Sur 20 personnes | |
|---|---|---|
| Très nette amélioration | 6 | 30% |
| Nette amélioration | 10 | 50% |
| Pas d'amélioration | 4 | 20% |
| Aggravation | 0 | 0% |

- Innocuité : Ce complément nutritionnel a été bien toléré. Au cours de toute la période de 60 jours d'ingestion, aucun évènement indésirable sérieux n'a été observé. Aucune anomalie a été observée pour les paramètres hépatiques (ASAT, ALAT, GGT), rénaux (urée, créatinine) et hématologiques (leucocytes, polynucléés, lymphocytes, monocytes, plaquettes), ce qui ont confirme l'innocuité de ce complément nutritionnel.

## Revendications

1. Procédé de préparation d'une fraction protéique laitière **caractérisé en ce qu'**il comprend les étapes suivantes :
le produit de départ est du lait ou du lactosérum ;
(a) le lait ou le lactosérum est éventuellement soumis à une microfiltration ou à un traitement thermique ;
(b) le lait ou le lactosérum ou le produit issu de l'étape (a) est déposé sur une résine échangeuse de cations constituée d'un polymère synthétique nanoporeux fonctionnalisé des groupes fonctionnels de type base conjuguée d'un anion d'un acide fort ; la vitesse linéaire est comprise entre 2,8 et 4,5 m/h et le volume de lait ou de lactoserum par rapport au volume de résine est compris entre 100 et 200 ;
(c) la résine échangeuse de cations est lavée par de l'eau déminéralisée ;
(d) la résine est éluée par des solutions de saumure de concentration croissante ;
(e) un éluat correspondant à une solution de saumure de conductivité comprise entre 21,0 et 22,0 mS/cm est récupéré.

2. Procédé selon la revendication 1, **caractérisé en ce qu'**il comporte en outre une ou plusieurs des étapes suivantes :
(f) l'éluat obtenu en (e) est concentré par ultrafiltration et on récupère le rétentat ;
(g) le rétentat obtenu en (f) est stérilisé par microfiltration et on récupère le perméat ;
(h) le perméat obtenu en (g) est séché par atomisation.

3. Procédé selon la revendication 1 ou la revendication 2, **caractérisé en ce que** le produit de départ est du lait de vache.

4. Procédé selon l'une quelconque des revendications 1 à 3, **caractérisé en ce que** ledit polymère de la résine échangeuse de cations est fonctionnalisé par des groupes SO₃⁻.

5. Procédé selon l'une quelconque des revendications précédentes, **caractérisé en ce que** le débit d'alimentation de la colonne à l'étape (b) est compris entre 10 et 15 m³/h.

6. Procédé selon l'une quelconque des revendications précédentes, **caractérisé en ce que** l'une ou plusieurs des conditions suivantes est vérifée :
- la solution de saumure comprend une concentration de sel de 10 à 14 g/l ;
- le débit de saumure est de 1,5 à 2 m³/h ;
- la vitesse linéaire de la solution de saumure est de 0,4 à 0,6 m/h ;
- le volume de saumure par rapport au volume de résine est compris entre 2,5 et 3,5.

7. Fraction protéique laitière susceptible d'être obtenue par le procédé selon l'une quelconque des revendications précédentes, **caractérisée en ce qu'**elle vérifie les caractéristiques suivantes :
- teneur en TGF-β comprise entre 0,010 et 0,025 % en poids par rapport au poids total des protéines ;
- teneur en IgG (Immuno Globuline) < 25 % en poids par rapport au poids total des protéines ;
- ratio TGF-β / IGF 1 ≥ 5 en poids/poids ;
- teneur en lactoperoxydase comprise entre 35 et 45 % en poids par rapport au poids total des protéines.

8. Utilisation d'une fraction protéique laitière selon la revendication 7 pour la préparation d'un médicament destiné à la prévention ou au traitement des pathologies inflammatoires chroniques et/ou de leurs symptômes.

9. Utilisation selon la revendication 8 pour la préparation d'un médicament destiné à la prévention ou au traitement du psoriasis et/ou de ses symptômes.

10. Utilisation d'une fraction protéique laitière selon la revendication 7 pour la préparation d'un médicament destiné à la prévention ou au traitement des maladies auto-immunes.

11. Utilisation d'une fraction protéique laitière selon la revendication 7 pour la préparation d'un médicament destiné à la prévention ou au traitement des manifestations inflammatoires des maladies auto-immunes.

12. Composition pharmaceutique comprenant une fraction protéique laitière selon la revendication 7 et un support pharmaceutiquement acceptable.

13. Composition alimentaire, notamment composition diététique, comprenant une fraction protéique laitière selon la revendication 7.

14. Procédé selon l'une quelconque des revendications 1 à 6, **caractérisé en ce qu'**il comporte en outre une étape (e') d'isolement d'une fraction protéique laitière enrichie en lactoferrine dans laquelle un éluat correspondant à une solution de saumure de conductivité comprise entre 50,5 et 51,5 mS/cm est récupéré.

15. Procédé selon la revendication 14, **caractérisé en ce que** l'étape (e') du procédé est mise en oeuvre en utilisant une ou plusieurs des conditions suivantes :
- volume de saumure par rapport au volume de résine compris entre 2,5 et 3,5,
- débit de saumure compris entre 1,5 et 2 m³/h,
- vitesse linéaire d'élution comprises entre 0,4 et 0,6 m/h.

## Patentansprüche

1. Verfahren zum Herstellen einer Milchproteinfraktion, dadurch gekennzeichet, dass es die nachfolgenden Schritte umfasst,
wobei das Ausgangsprodukt Milch oder Molke ist:
(a) die Milch oder Molke gegebenenfalls einer Mikrofiltration oder einer Wärmebehandlung unverzogen wird;
(b) die Milch oder Molke oder das aus Schritt (a) erhaltene Produkt auf ein Kationenaustauschharz aufgebracht wird, das aus einem nanoporösen, synthetischen Polymer besteht, funktionalisiert mit funktionellen Gruppen basischer Art, konjugiert mit einem Anion einer starken Säure, wobei die lineare Geschwindigkeit zwischen 2,8 und 4,5 m/h und das Milch- oder Molkevolumen bezogen auf das Harzvolumen zwischen 100 und 200 liegt;
(c) das Kationenaustauschharz mit demineralisiertem Wasser gespült wird;
(d) das Harz mit Salzlösungen steigender Konzentration eluiert wird;
(e) ein Eluat gewonnen wird, das einer Salzlösung mit einer Leitfähigkeit von zwischen 21,0 und 22,0 mS/cm entspricht.

2. Verfahren nach Anspruch 1, **dadurch gekennzeichnet, dass** es ferner einen oder mehrere der nachfolgenden Schritte umfasst:
(f) das in (e) erhaltene Eluat durch Ultrafiltration konzentriert und das Retentat gewonnen wird;
(g) das in (f) erhaltene Retentat durch Mikrofiltration sterilisiert und das Permeat gewonnen wird;
(h) das in (g) erhaltene Permeat durch Zerstäubung getrocknet wird.

3. Verfahren nach Anspruch 1 oder Anspruch 2, **dadurch gekennzeichnet, dass** das Ausgangsprodukt Kuhmilch ist.

4. Verfahren nach einem der Ansprüche 1 bis 3, **dadurch gekennzeichnet, dass** das Polymer des Kationenaustauschharzes mit SO₃⁻-Gruppen funktionalisiert wird.

5. Verfahren nach einem der vorangehenden Ansprüche, **dadurch gekennzeichnet, dass** der Durchsatz der Kolonne in Schritt (b) zwischen 10 und 15 m³/h beträgt.

6. Verfahren nach einem der vorangehenden Ansprüche, **dadurch gekennzeichnet, dass** eine oder mehrere der nachfolgenden Bedingungen erfüllt sind:
- die Salzlösung weist eine Salzkonzentration von 10 bis 14 g/l auf;
- der Durchsatz der Salzlösung beträgt 1,5 bis 2 m³/h;
- die lineare Geschwindigkeit der Salzlösung beträgt 0,4 bis 0,6 m/h;
- das Salzlösungsvolumen bezogen auf das Harzvolumen liegt zwischen 2,5 und 3,5.

7. Milchproteinfraktion erhältlich mit dem Verfahren nach einem der vorangehenden Ansprüche, **dadurch gekennzeichnet, dass** sie den nachfolgendes Merkmalen entspricht:
- Gehalt an TGF-β zwischen 0,010 und 0,025 Gew.-% bezogen auf das Gesamtgewicht der Proteine;
- Gehalt an IgG (Immunglobulin) < 25 Gew.-% bezogen auf das Gesamtgewicht der Proteine;
- Gewichtsverhältnis TGF-β / IGF 1 ≥ 5;
- Gehalt an Lactoperoxidase liegt zwischen 35 und 45 Gew.-% bezogen auf das Gesamtgewicht der Proteine.

8. Verwendung einer Milchproteinfraktion nach Anspruch 7 zum Herstellen eines Arzneimittels, das zur Vorbeugung oder Behandlung von chronische Entzündungserkrankungen und/oder deren Symptome bestimmt ist.

9. Verwendung nach Anspruch 8 zum Herstellen eines Arzneimittels, das zur Vorbeugung oder Behandlung von Psoriasis und/oder deren Symptome bestimmt ist.

10. Verwendung einer Milchproteinfraktion nach Anspruch 7 zum Herstellen eines Arzneimittels, das zur Vorbeugung oder Behandlung von Autoimmunerkrankungen bestimmt ist.

11. Verwendung einer Milchproteinfraktion nach Anspruch 7 zum Herstellen eines Arzneimittels, das zur Vorbeugung oder Behandlung von Entzündungserscheinungen von Autoimmunerkrankungen bestimmt ist.

12. Pharmazeutische Zusammensetzung umfassend eine Milchproteinfraktion nach Anspruch 7 und einen pharmazeutisch verträglichen Träger.

13. Nahrungsmittelzusammensetzung, insbesondere diätetische Zusammensetzung umfassend eine Milchproteinfraktion nach Anspruch 7.

14. Verfahren nach einem der Ansprüche 1 bis 6, **dadurch gekennzeichnet, dass** es ferner einen Schritt (e') der Isolierung einer mit Lactoferrin angereicherten Milchproteinfraktion umfasst, bei welchem ein Eluat gewonnen wird, das einer Salzlösung mit einer Leitfähigkeit von zwischen 50,5 und 51,5 mS/cm entspricht.

15. Verfahren nach Anspruch 14, **dadurch gekennzeichnet, dass** der Schritt (e') des Verfahrens unter Anwendung einer oder mehrerer der nachfolgenden Bedingungen durchgeführt wird:
- Salzlösungsvolumen bezogen auf das Harzvolumen liegt zwischen 2,5 und 3,5,
- Durchsatz der Salzlösung liegt zwischen 1,5 und 2 m³/h,
- lineare Geschwindigkeit der Elution liegt zwischen 0,4 bis 0,6 m/h.

## Claims

1. A process for preparing a milk protein fraction comprising the following steps:
milk or whey are used as starting material;
(a) the milk or whey is optionally subjected to a microfiltration or to a heat treatment;
(b) the milk or whey or the product derived from step (a) is deposited on a cation-exchange resin consisting in a nanoporous synthetic polymer functionalized with functional groups of the conjugate base type of an anion of a strong acid; the linear speed is between 2.8 and 4.5 m/h and the volume of milk relative to the volume of resin is between 100 and 200;
(c) the cation-exchange resin is washed with demineralized water;
(d) the resin is eluted with brine solutions of increasing concentration;
(e) an eluate corresponding to a brine solution with a conductivity of between 21.0 and 22.0 mS/cm is recovered.

2. The process as claimed in claim 1 additionally comprising one or more of the following steps:
(f) the eluate obtained in (e) is concentrated by ultrafiltration and the retentate is recovered;
(g) the retentate obtained in (f) is sterilized by microfiltration and the permeate is recovered;
(h) the permeate obtained in (g) is spray-dried.

3. The process as claimed in claim 1 or in claim 2 wherein the starting material is cow's milk.

4. The process as claimed in claims 1 to 3, wherein the polymer is functionalized with SO₃⁻ groups.

5. The process as claimed in anyone of the preceding claims, wherein the flow rate for supplying the column in step (b) is between 10 and 15 m³/h.

6. The process as claimed in anyone of the preceding claims, wherein one or more of the following conditions are satisfied:
- the brine solution comprises a salt concentration of 10 to 14 g/l;
- the brine flow rate is 1.5 to 2 m³/h;
- the linear speed of the brine solution is from 0.4 to 0.6 m/h;
- the volume of brine relative to the volume of resin is between 2.5 and 3.5.

7. A milk protein fraction obtainable by the process according to anyone of the preceding claims which satisfies the following characteristics:
- TGF-β content of between 0.010 and 0.025% by weight relative to the total weight of proteins;
- IgG (Immunoglobulin) content <25% by weight relative to the total weight of proteins;
- TGF- β/IGF 1 ratio ≥ 5 by weight/weight;
- lactoperoxidase content between 35 and 45% by weight relative to the total weight of proteins.

8. Milk protein fraction according to claim 7 for use in the prevention and/or treatment of chronic inflammatory pathological conditions and/or their symptoms.

9. Milk protein fraction according to claim 7 for its use according to claim 8 for the prevention and/or treatment of psoriasis and/or its symptoms.

10. Milk protein fraction according to claim 7 for its use according to claim 8 for the prevention and/or treatment of the autoimmune diseases.

11. Milk protein fraction according to claim 7 for its use according to claim 8 for the prevention and/or treatment of the inflammatory manifestations of autoimmune diseases

12. A pharmaceutical composition comprising a milk protein fraction as claimed in claim 7 and a pharmaceutically acceptable carrier.

13. A food composition comprising a milk protein fraction as claimed in claim 7.

14. The process as claimed in anyone of claims 1 to 6 additionally comprising a step (e') of isolating a lactoferrin-enriched milk protein fraction in which an eluate corresponding to a brine solution having a conductivity between 50.5 and 51.5 mS/cm is recovered.

15. The process as claimed in claim 14, wherein step (e') of the process is carried out using one or more of the following conditions:
- volume of brine relative to the volume of resin between 2.5 and 3.5,
- flow rate of brine between 1.5 and 2 m³/h,
- linear speed of elution between 0.4 and 0.6 m/h.
